# EUROPÄISCHE PATENTANMELDUNG

(11) **EP 1 469 201 A2**
(43) Veröffentlichungstag der Anmeldung: **20.10.2004**
(21) Anmeldenummer: 04002980.3
(22) Anmeldetag: 11.02.2004
(51) Int. Cl.: F04B 43/12

(54) **Medizinische Schlauchpumpe**

(30) Priorität: 17.04.2003 DE 20306163 U
(71) Anmelder: Richard Wolf GmbH, 75438 Knittlingen (DE)
(72) Erfinder: Wehrheim, Frank, 64319 Pfungstadt (DE)
(74) Vertreter: Hemmer, Arnd, Dipl.-Ing.

(57) **Zusammenfassung**

Die Erfindung betrifft eine medizinische Schlauchpumpe mit einem Pumpenrad (6) und zwei Schlaucheinlegestellen (20, 22), in welche ein Pumpschlauch derart einlegbar ist, dass er das Pumpenrad (6) umschlingt, wobei das Pumpenrad (6) an seiner Frontseite an seinem gesamten Umfang von einem feststehenden Schutzring (12) zur Abdeckung des Pumpschlauchs umgeben ist (Fig. 1).

## Beschreibung

Die Erfindung betrifft eine medizinische Schlauchpumpe.

Derartige Schlauchpumpen bzw. Schlauchrollenpumpen werden in der Medizintechnik eingesetzt, um verschiedene Flüssigkeiten zu einem Patienten zu- und/oder abzuführen. Diese Pumpen weisen ein Pumpenrad auf, an dessen Umfang Nocken oder Rollen angeordnet sind. Ein Pumpschlauch umschlingt das Pumpenrad, so dass der Schlauch umlaufend durch die Nocken bzw. Rollen bei Drehung des Pumpenrades gequetscht wird, wodurch eine Flüssigkeit im Inneren des Pumpschlauches weitertransportiert wird. Derartige Schlauchpumpen werden als offene oder geschlossene Schlauchpumpen ausgebildet. Bei der geschlossenen Ausbildung ist das Pumpenrad unter einem Deckel angeordnet, so dass Pumpenrad und eingelegter Schlauch im Inneren eines Gehäuses angeordnet sind. Bei diesen Pumpen ist sichergestellt, dass ein Betrieb nur bei geschlossenem Deckel möglich ist, so dass Verletzungsgefahren an dem Pumpenrad ausgeschlossen sind. Allerdings ist der Aufbau dieser Schlauchpumpen aufwändiger und das Einlegen des Pumpschlauches komplizierter als bei den offen ausgebildeten Schlauchpumpen. Bei diesen ist das Pumpenrad außerhalb des Gehäuses, beispielsweise an der Frontplatte des Pumpengehäuses vorgesehen. Seitlich des Pumpenrades sind ferner zwei Schlaucheinlegestellen vorgesehen, in denen der Pumpschlauch so fixiert wird, dass er das Pumpenrad in einem Winkel von im Wesentlichen 180° umschlingt. Diese Anordnung hat den Nachteil, dass insbesondere bei falsch eingelegtem Pumpschlauch ein Verletzungsrisiko derart besteht, dass beispielsweise Finger zwischen Pumpschlauch und Pumpenrad eingeklemmt oder gequetscht werden können.

Es ist Aufgabe der Erfindung, eine verbesserte medizinische Schlauchpumpe bereitzustellen, welche auch bei offener Ausgestaltung einen ausreichenden Schutz vor Verletzungen bietet. Diese Aufgabe wird durch eine medizinische Schlauchpumpe mit den im Anspruch 1 angegebenen Merkmalen gelöst. Bevorzugte Ausführungsformen ergeben sich aus den Unteransprüchen.

Die erfindungsgemäße medizinische Schlauchpumpe weist ein Pumpenrad und zwei Schlaucheinlegestellen auf, in welche ein Pumpschlauch derart einlegbar ist, dass er das Pumpenrad umschlingt. Dabei sind die Schlaucheinlegestellen in bekannter Weise seitlich des Pumpenrades angeordnet, so dass ein in den Schlaucheinlegestellen fixierter Pumpschlauch das Pumpenrad über einen Winkelbereich von etwa 180° umschlingen kann. Das Pumpenrad kann in bekannter Weise Rollen oder Nocken aufweisen, welche bei Drehung den Schlauch zur Förderung eines Fluids umlaufend quetschen. Zum Schutz vor Verletzungen ist bei der erfindungsgemäßen Schlauchpumpe ein Schutzring vorgesehen, welcher das Pumpenrad entlang seines gesamten Umfanges umgibt. Dabei ist der Schutzring so angeordnet, dass er im Wesentlichen in der Ebene der Frontseite bzw. Oberseite des Pumpenrades angeordnet ist. Bei einer offen ausgestalteten Schlauchpumpe entsteht somit ein Spalt zwischen dem Schutzring und dem Pumpengehäuse, in welchen der Pumpschlauch eingelegt wird. Nach außen hin wird der Pumpschlauch dabei durch den Schutzring abgedeckt, so dass Klemmungen oder Quetschungen zwischen Schlauch und Pumpenrad verhindert werden können. Der Schutzring erstreckt sich am Umfang des Pumpenrades von diesem radial nach außen und bildet somit im Wesentlichen eine radiale Verlängerung der Frontfläche des Pumpenrades. Dabei ist der Schutzring feststehend ausgebildet, d. h. er dreht sich nicht gemeinsam mit dem Pumpenrad. Der Schutzring ist bevorzugt fest an dem Pumpengehäuse angebracht. Der Schutzring überdeckt die Bereiche zwischen dem sich drehenden Pumpenrad und dem festliegenden, in den Schlaucheinlegestellen fixierten Pumpschlauch, in denen die Gefahr von Quetschungen besteht. Zwischen dem Schutzring und dem Pumpschlauch besteht keine Gefahr von Quetschungen, da diese beide feststehend sind, so dass keine Relativbewegung zwischen Schutzring und Pumpschlauch stattfindet.

Bevorzugt grenzt der Schutzring über den gesamten Umfang des Pumpenrades berührungsfrei direkt an das Pumpenrad an. Das heißt, der Spalt zwischen feststehendem Schutzring und sich drehendem Pumpenrad ist möglichst schmal ausgebildet, so dass ein Einklemmen zwischen Pumpenrad und Schutzring unmöglich wird. Allerdings muss ein schmaler Spalt zwischen Pumpenrad und Schutzring bestehen, um eine reibungsfreie Drehung des Pumpenrades relativ zu dem Schutzring zu ermöglichen.

Bevorzugt erstreckt sich der Schutzring im Wesentlichen bündig zu der Frontseite des Pumpenrades. Das bedeutet, die Oberfläche des Schutzringes liegt mit der Oberfläche der Stirn- bzw. Frontseite des Pumpenrades in einer Ebene, so dass eine glatte Frontfläche geschaffen wird. Die glatte Ausgestaltung von Pumpenrad und Schutzring verhindert ein Verhaken oder Verklemmen von Gegenständen an dem sich bewegenden Pumpenrad.

Weiter bevorzugt wird der Schutzring von einer Frontplatte gebildet, welche die Frontseite des Pumpenrades überdeckt uns sich über den Umfang des Pumpenrades radial nach außen erstreckt. Die Frontplatte ist dabei vor dem Pumpenrad beabstandet zu der Front- bzw. Stirnfläche des Pumpenrades angeordnet, so dass sich das Pumpenrad berührungsfrei unter der Frontplatte drehen kann. Der Abstand zwischen Frontplatte und Stirnseite des Pumpenrades ist möglichst gering ausgebildet, so dass es nicht möglich ist, dass sich der Pumpschlauch zwischen Pumpenrad und Frontplatte verklemmen kann. Die Frontplatte erstreckt sich radial über den Umfang des Pumpenrades hinaus, so dass die Frontplatte einen eingelegten Pumpschlauch zumindest teilweise überdeckt. Dabei wird zumindest der Bereich des Pumpschlauches, welcher in Kontakt mit dem Pumpenrad tritt, überdeckt, so dass die Gefahr eines Einklemmens von Gegenständen oder Körperteilen zwischen Pumpschlauch und Pumpenrad verhindert wird. Die Ausführungsform, bei welcher der Schutzring, d. h. derjenige Teil, welcher sich über den Umfang des Pumpenrades radial nach außen erstreckt, Teil einer durchgängigen Frontplatte ist, hat den Vorteil, dass keinerlei bewegte Bauteile offen liegen, so dass eine Verletzungsgefahr verringert wird. Allerdings bleibt der seitliche Bereich des Pumpenrades unterhalb der Frontplatte offen, so dass der Pumpschlauch problemlos eingelegt werden kann, ohne dass irgendwelche Abdeckungen geöffnet werden müssen.

Bevorzugt bilden der Schutzring und die Schlaucheinlegestellen eine Baugruppe. Das heißt, die Schlaucheinlegestellen werden gemeinsam mit dem Schutzring als ein Bauteil bereitgestellt, welches beispielsweise an die Frontplatte eines Pumpengehäuses angesetzt werden kann. Auf diese Weise wird die Anzahl der erforderlichen Bauteile reduziert und die Montage vereinfacht.

Weiter bevorzugt ist der Schutzring bzw. die Frontplatte einstückig mit den Schlaucheinlegestellen vorzugsweise aus Kunststoff ausgebildet. Ein solches Bauteil kann beispielsweise als Spritzgussteil sehr kostengünstig hergestellt werden. In diesem einstückigen Bauteil sind entsprechende Ausnehmungen ausgeformt, welche als Schlaucheinlegestellen dienen. Ferner weist das Bauteil eine kreisförmige Durchgangsöffnung auf, deren umgebendes Material den Schutzring bildet. In die Durchgangsöffnung greift bei montiertem Schutzring das Pumpenrad ein.

Der Schutzring ist bevorzugt Teil eines das Pumpenrad umgebenden Käfigs. Dieser Käfig umgibt den gesamten umfänglichen Bereich des Pumpenrades und dient somit als Schutz vor Verletzungen beispielsweise durch Einklemmen an den bewegten Teilen, d. h. dem Pumpenrad.

Der Käfig weist vorzugsweise einen hinteren Ring auf, welcher das Pumpenrad umfänglich umgibt und an einer Frontplatte eines Pumpengehäuses anliegt, wobei der hintere Ring von dem Schutzring um ein Maß beabstandet ist, welches gleich oder größer als der Durchmesser des aufzunehmenden Pumpschlauches ist. Diese Anordnung ermöglicht, dass der Pumpschlauch zwischen dem Schutzring und dem hinteren Ring eingelegt werden kann und durch diese beiden Ringe fixiert bzw. positioniert wird. Ferner werden durch den Schutzring und den hinteren Ring die entstehenden Spalte zwischen Pumpschlauch und bewegtem Pumpenrad abgedeckt, so dass in diesen Bereichen ein Einklemmen zwischen bewegten und feststehenden Teilen nicht möglich ist.

Vorzugsweise ist zwischen dem Schutzring und dem hinteren Ring in einem zur Aufnahme eines Pumpschlauches bestimmten Umfangsabschnitt ein in radialer Richtung durchgängiger Spalt ausgebildet. Das heißt, in dem Bereich, in dem der Pumpschlauch das Pumpenrad umschlingt, ist der dem Pumpenrad zugewandte Raum zwischen dem Schutzring und dem hinteren Ring offen ausgebildet, so dass der Pumpschlauch in diesem Bereich mit den Rollen bzw. Nocken an dem Pumpenrad in Kontakt kommen kann. In diesem Bereich sind keinerlei Verbindungen zwischen dem Schutzring und dem hinteren Ring vorgesehen, so dass über diesen gesamten Umfangabschnitt das Pumpenrad mit dem Pumpschlauch in Kontakt kommen kann. Ferner ist der Spalt zwischen Schutzring und hinterem Ring, in den der Pumpschlauch eingelegt wird, über den Umfangsbereich zwischen den Schlaucheinlegestellen des Käfigs offen ausgebildet, so dass der Pumpschlauch einfach von außen eingelegt werden kann, ohne dass Bauteile geöffnet oder entfernt werden müssen.

Der Schutzring und der hintere Ring des Käfigs sind zweckmäßigerweise im Bereich der Schlaucheinlegestellen miteinander verbunden. Auf diese Weise wird ein einteiliges, vorzugsweise einstückiges Bauteil bestehend aus Schutzring, hinterem Ring und den Schlaucheinlegestellen geschaffen. Dieses einteilige Bauteil kann leicht an einem Pumpengehäuse, insbesondere der Frontplatte eines Pumpengehäuses angebracht werden. Im Bereich der Schlaucheinlegestellen können der Schutzring und der hintere Ring miteinander verbunden sein, ohne dass das Einlegen des Pumpschlauches behindert würde, da somit im übrigen Umfangsbereich, in dem der Pumpschlauch das Pumpenrad umschlingen soll, ein in radialer Richtung durchgehender, nach außen geöffneter Spalt zur Aufnahme des Pumpenschlauches ausgebildet werden kann.

Gemäß einer besonderen Ausführungsform weist zumindest eine Schlaucheinlegestelle eine Aufnahme für ein Sensorsystem auf. So kann im Bereich zumindest einer Schlaucheinlegestelle eine vergrößerte Aufnahme bzw. Ausnehmung ausgebildet sein, in welche eine Druckkammer eines Drucksensors eingesetzt werden kann. Die Druckkammer wird dabei vorzugsweise durch Klemmen und/oder Verrasten in der Ausnehmung kraft- und/oder formschlüssig fixiert. Dies ermöglicht eine einfache Montage und Fixierung der Druckkammer bzw. eines Sensorsystems.

Der Schutzring weist vorzugsweise eine radiale Ausdehnung auf, welche zumindest dem Durchmesser des Pumpschlauches entspricht. Dadurch wird sichergestellt, dass der Pumpschlauch weitgehend durch den Schutzring überdeckt wird und insbesondere der entstehende Spalt zwischen festliegendem Pumpschlauch und sich drehendem Pumpenrad vollständig durch den Schutzring überdeckt ist, so dass in diesem Bereich keine Verletzungsgefahr mehr gegeben ist.

Die oben beschriebene Anordnung eines Schutzringes ermöglicht eine bessere Führung und Fixierung des Pumpschlauches an dem Pumpenrad. Gleichzeitig werden die Bereiche, in denen eine erhöhte Verletzungsgefahr besteht, durch den Schutzring abgedeckt, so dass ein sichererer Betrieb der erfindungsgemäßen Schlauchpumpe möglich ist.

Nachfolgend wir die Erfindung beispielhaft anhand der beigefügten Figuren beschrieben.

In diesen zeigt
- Fig. 1: eine perspektivische Ansicht einer Frontplatte eines Pumpengehäuses,
- Fig. 2: eine perspektivische Ansicht des erfindungsgemäßen Schutzkäfigs.

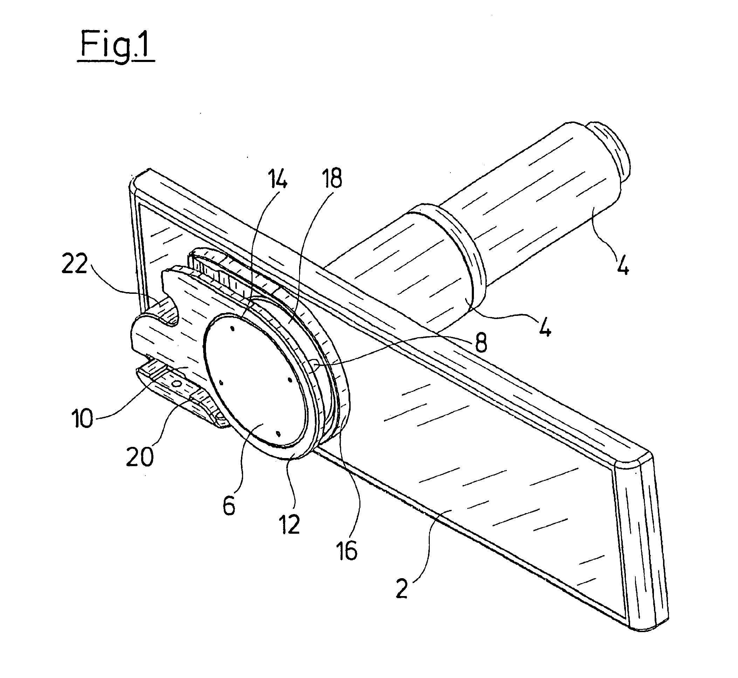

Fig. 1 zeigt in einer perspektivischen Ansicht schematisch die Frontplatte 2 eines Pumpengehäuses. An der Rückseite der Frontplatte 2, d. h. im Inneren des Pumpengehäuses (hier nicht gezeigt) ist eine Antriebseinheit 4 angeordnet. Die Antriebseinheit 4 weist beispielsweise einen Elektromotor mit zugehörigen Getriebeeinrichtungen auf. An der Frontseite der Frontplatte 2 ist das Pumpenrad 6 angeordnet. Das Pumpenrad 6 wird von der Antriebseinheit 4 drehbar angetrieben. Am Umfang des Pumpenrades 6, welches im vorliegenden Fall als Rollenrad ausgestaltet ist, sind einzelne Rollen 8 angeordnet. Die Rollen 8 laufen bei Drehung des Pumpenrades 6 in bekannter Weise über einen eingelegten Pumpschlauch (hier nicht gezeigt) und bewirken somit einen Pumpeffekt in dem Schlauch.

Erfindungsgemäß ist das Pumpenrad 6 von einem Schutzkäfig bzw. Käfig 10 umgeben. Der Schutzkäfig 10 weist an seiner Vorderseite, d. h. an seiner von der Frontplatte 2 abgewandten Seite einen Schutzring 12 auf, welcher das Pumpenrad 6 an dessen Front- bzw. Stirnfläche umfänglich umgibt. Dabei bildet die Frontfläche des Käfigs 10 und des Schutzringes 12 im Wesentlichen eine Ebene mit der Frontfläche des Pumpenrades 6. Der umfängliche Spalt 14 zwischen Pumpenrad 6 und Schutzring 12 ist möglichst schmal ausgebildet, damit sich keine Gegenstände oder Körperteile in dem Spalt 14 zwischen bewegbarem Pumpenrad 6 und feststehendem Schutzring 12 verfangen bzw. einklemmen können.

Der Käfig 10 weist ferner einen hinteren Ring 16 auf, welcher das Pumpenrad 6 an seiner der Frontplatte 2 zugewandten Seite umfänglich umgibt. Der Schutzring 12 und der hintere Ring 16 sind in der Richtung normal zu der Frontplatte 2 voneinander beabstandet angeordnet, so dass ein Spalt 18 zwischen Schutzring 12 und hinterem Ring 16 gebildet wird. Der Spalt 18 ist am Umfang des Pumpenrades 6 offen ausgebildet, so dass ein Pumpschlauch (hier nicht gezeigt) von außen eingelegt werden kann. Dabei ist der Spalt 18 in einem Umfangsbereich von 180°, in dem das Pumpenrad 6 von einem Pumpschlauch umschlungen werden kann, als in radialer Richtung durchgehender Spalt ausgebildet. Das bedeutet, dass in diesem Bereich keinerlei Verbindungen zwischen hinterem Ring 16 und Schutzring 12 vorgesehen sind, so dass ein Pumpschlauch in diesem Bereich über den gesamten Umfangsabschnitt des Pumpenrades 6 mit diesen in Kontakt treten kann. Ferner erstreckt sich der Spalt 18 ausgehend von dem Umschlingungsbereich am Umfang des Pumpenrades 6 tangential bis zu den Schlaucheinlegestellen 20 und 22.

Die Schlaucheinlegestellen 20 und 22 sind einstückig in dem Käfig 10 als Aufnahmen bzw. Ausnehmungen ausgebildet.

Die Schlaucheinlegestelle 20 ist als Aufnahme für ein Sensorsystem beispielsweise in Form einer Druckkammer ausgebildet. Die Schlaucheinlegestelle 20 ist nach vorne, d. h. zur Frontseite des Käfigs 10 hin geöffnet, wobei diese Öffnung in Verbindung mit dem Spalt 18 steht, so dass der Käfig 10 am gesamten Umfang des Pumpenrades 6 zur Aufnahme eines Pumpschlauches geöffnet ausgebildet ist.

Das Einlegen des Pumpschlauches (in Fig. 1 nicht gezeigt) in die Schlauchpumpe mit dem erfindungsgemäßen Käfig 10 erfolgt in der Weise, dass der Pumpschlauch mit einer Druckkammer in die erste Schlaucheinlegestelle 20 von vorne, d. h. in einer Richtung normal zu der Frontplatte 2, eingesetzt wird, wobei der Schlauch bzw. die Druckkammer kraft- und/oder formschlüssig in der Schlaucheinlegestelle 20 fixiert wird. In der Schlaucheinlegestelle 20 sind Vorsprünge ausgebildet, welche von der mit dem Schlauch verbundenen Druckkammer hintergriffen werden, so dass eine Kraftübertragung in Längsrichtung des Pumpschlauches möglich ist. Anschließend wird der Pumpschlauch weiter in den Spalt 18 eingelegt und an der zweiten Schlaucheinlegestelle 22 fixiert. Dazu ist an dem Pumpschlauch ein entsprechendes Befestigungselement ausgebildet, welches in die Schlaucheinlegestelle 22 eingreift, so dass der Pumpschlauch zwischen den Schlaucheinlegestellen 20 und 22 unter Vorspannung derart fixiert wird, dass er in dem Spalt 18 das Pumpenrad 6 umschlingt.

Durch den Schutzring 12 wird der ein hohes Verletzungsrisiko beinhaltende Bereich zwischen dem festliegenden Pumpschlauch und dem sich drehenden Pumpenrad 6 schützend überdeckt. Der hintere Ring 16 überdeckt den Bereich zwischen Frontplatte 2 und Pumpschlauch, so dass auch an dieser Stelle das Verletzungsrisiko minimiert wird. Gleichzeitig dienen der Schutzring 12 und der hintere Ring 16 zur Führung des das Pumpenrad 6 umschlingenden Pumpschlauches, so dass ein fehlerhaftes Einlegen des Pumpschlauches und ein damit verbundenes Verletzungs- oder Beschädigungsrisiko weitgehend ausgeschlossen wird. Insgesamt wird durch den Käfig 10 somit ein sicherer Betrieb einer offen ausgebildeten Schlauchpumpe gewährleistet.

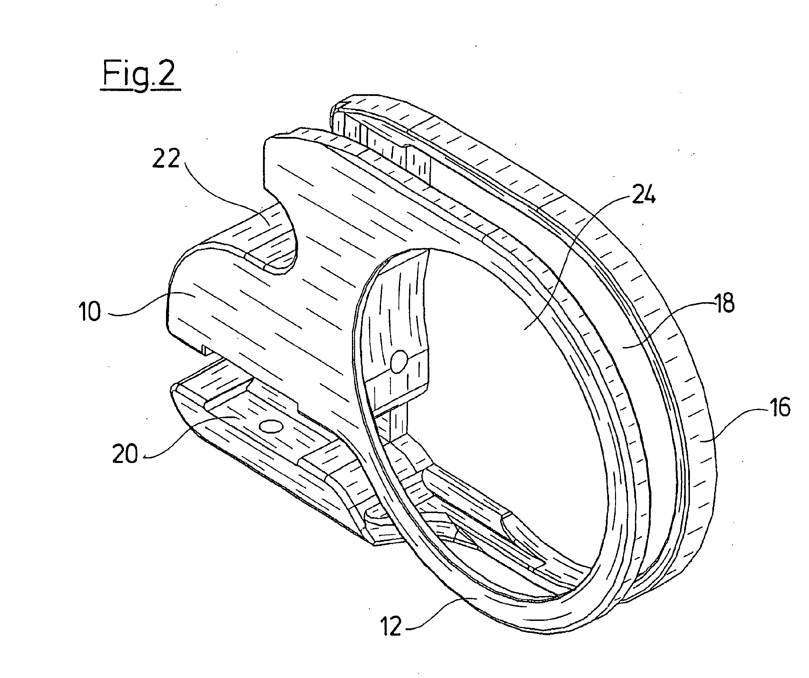

Fig. 2 zeigt eine perspektivische Detailansicht des Käfigs 10. Ausgehend von dem Bereich des Käfigs 10, in dem die Schlaucheinlegestellen bzw. Schlauchlagerstellen 20 und 22 ausgebildet sind, erstrecken sich der Schutzring 12 sowie der hintere Ring 16 parallel beabstandet zueinander. Der Schutzring 12 und der hintere Ring 16 umschließen eine Ausnehmung in Form eines kreisförmigen Durchgangsloches 24, welches zur Aufnahme des Pumpenrades 6 bestimmt ist. Zwischen dem hinteren Ring 16 und dem Schutzring 12 verläuft der durchgehende Spalt 18, welcher zu dem Loch 24 und nach außen hin geöffnet ist. Der Spalt 18 erstreckt sich nach außen geöffnet bis in die Schlauchlagerstelle 22 hinein. Die Schlauchlagerstelle 20 weist eine zur Frontfläche hin geöffnete Nut auf, welche mit dem Spalt 18 in Verbindung steht. Die nach außen gerichteten Öffnungen der Nut der Schlaucheinlegestelle 20 und des Spaltes 18 sind somit um 90° versetzt zueinander angeordnet. Die durchgehende Öffnung des Spaltes 18 ermöglicht ein einfaches Einlegen eines Pumpschlauches von der Umfangsseite des Pumpenrades 6 her, ohne dass irgendwelche Elemente geöffnet oder abgenommen werden müssen. Der in Fig. 2 gezeigte Käfig 10 ist, wie in Fig. 1 gezeigt, fest, d. h. drehfest mit der Frontplatte 2 verbunden, so dass der Käfig 10 einen eingelegten Pumpschlauch fest an der Frontplatte 2 hält, während das Pumpenrad 6 sich relativ zu dem eingelegten Pumpschlauch dreht.

### Bezugszeichenliste

- 2 -: Frontplatte
- 4 -: Antriebseinheit
- 6 -: Pumpenrad
- 8 -: Rolle
- 10 -: Käfig
- 12 -: Schutzring
- 14 -: Spalt
- 16 -: hinterer Ring
- 18 -: Spalt
- 20, 22 -: Schlaucheinlegestellen
- 24 -: Ausnehmung

## Patentansprüche

1. Medizinische Schlauchpumpe mit einem Pumpenrad (6) und zwei Schlaucheinlegestellen (20, 22), in welche ein Pumpschlauch derart einlegbar ist, dass er das Pumpenrad (6) umschlingt, wobei das Pumpenrad (6) an seiner Frontseite an seinem gesamten Umfang von einem feststehenden Schutzring (12) zur Abdeckung des Pumpschlauchs umgeben ist.

2. Medizinische Schlauchpumpe nach Anspruch 1, bei welcher der Schutzring (12) über den gesamten Umfang des Pumpenrades (6) berührungsfrei direkt an das Pumpenrad (6) angrenzt.

3. Medizinische Schlauchpumpe nach Anspruch 1 oder 2, bei welcher der Schutzring (12) sich im Wesentlichen bündig zu der Frontseite des Pumpenrades (6) erstreckt.

4. Medizinische Schlauchpumpe nach Anspruch 1, bei welcher der Schutzring von einer Frontplatte gebildet wird, welche die Frontseite des Pumpenrades (6) überdeckt und sich über den Umfang des Pumpenrades (6) radial nach außen erstreckt.

5. Medizinische Schlauchpumpe nach einem der vorangehenden Ansprüche, bei welcher der Schutzring (12) mit den Schlaucheinlegestellen (20, 22) eine Baugruppe bildet.

6. Medizinische Schlauchpumpe nach Anspruch 5, bei welcher der Schutzring (12) einstückig mit den Schlaucheinlegestellen (20, 22) vorzugsweise aus Kunststoff ausgebildet ist.

7. Medizinische Schlauchpumpe nach einem der vorangehenden Ansprüche, bei welcher der Schutzring (12) Teil eines das Pumpenrad umgebenden Käfigs (10) ist.

8. Medizinische Schlauchpumpe nach Anspruch 7, bei welcher der Käfig (10) einen hinteren Ring (16) aufweist, welcher das Pumpenrad (6) umfänglich umgibt und an einer Frontplatte (2) eines Pumpengehäuses anliegt, wobei der hintere Ring (16) von dem Schutzring (12) um ein Maß beabstandet ist, welches gleich oder größer als der Durchmesser des aufzunehmenden Pumpschlauches ist.

9. Medizinische Schlauchpumpe nach Anspruch 8, bei welcher zwischen dem Schutzring (12) und dem hinteren Ring (16) in einem zur Aufnahme eines Pumpschlauches bestimmten Umfangsabschnitt ein in radialer Richtung durchgängiger Spalt (18) ausgebildet ist.

10. Medizinische Schlauchpumpe nach Anspruch 8 oder 9, bei welcher der Schutzring (12) und der hintere Ring (16) des Käfigs (10) im Bereich der Schlaucheinlegestellen (20, 22) miteinander verbunden sind.

11. Medizinische Schlauchpumpe nach einem der vorangehenden Ansprüche, bei welcher zumindest eine Schlaucheinlegestelle (20) eine Aufnahme für ein Sensorsystem aufweist.

12. Medizinische Schlauchpumpe nach einem der vorangehenden Ansprüche, bei welcher der Schutzring (12) eine radiale Ausdehnung aufweist, welche zumindest dem Durchmesser des Pumpschlauches entspricht.
